(19) Europäisches Patentamt
European Patent Office
Office européen des brevets

(11) **EP 2 371 406 A1**

(12) **EUROPEAN PATENT APPLICATION**
published in accordance with Art. 153(4) EPC

(43) Date of publication:
**05.10.2011 Bulletin 2011/40**

(51) Int Cl.:
***A61M 5/28*** (2006.01)

(21) Application number: **09830433.0**

(22) Date of filing: **03.12.2009**

(86) International application number:
**PCT/JP2009/070285**

(87) International publication number:
**WO 2010/064667 (10.06.2010 Gazette 2010/23)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **03.12.2008 JP 2008308978
19.12.2008 JP 2008323575
09.01.2009 JP 2009003518**

(71) Applicants:
- **Denki Kagaku Kogyo Kabushiki Kaisha
Tokyo 103-8338 (JP)**
- **Taisei Kako Co., Ltd.
Kita-ku
Osaka-shi
Osaka 531-0072 (JP)**

(72) Inventors:
- **HIOKI, Kazutoshi
Itoigawa-shi
Niigata 949-0393 (JP)**

- **NOHMI, Kenji
Itoigawa-shi
Niigata 949-0393 (JP)**
- **MIYATA, Yoshiaki
Tokyo 103-8338 (JP)**
- **MATSUMOTO, Ippei
Ibaraki-shi
Osaka 567-0054 (JP)**
- **ASAHI, Norihiko
Ibaraki-shi
Osaka 567-0054 (JP)**

(74) Representative: **Gulde Hengelhaupt Ziebig & Schneider
Patentanwälte - Rechtsanwälte
Wallstraße 58/59
10179 Berlin (DE)**

(54) **SYRINGE**

(57)     Provided is a syringe with which it is possible to hold with greater stability a liquid inside the barrel while maintaining the slidability and air-tightness between the barrel and the gasket without requiring fixation of silicone oil, and which is excellent in terms of accuracy of visual inspection. The syringe has a resin barrel, a gasket slidably inserted inside the barrel, a plunger attached to the gasket, and a silicone film obtained by applying silicone oil having a kinematic viscosity of 500 to 10,000 cSt over the inner peripheral surface of the barrel in an amount of 5 to 50 $\mu$g per 1 cm$^2$ of area.

Fig. 1

EP 2 371 406 A1

## Description

### TECHNICAL FIELD

The present invention relates to a syringe, and more particularly relates to a syringe which is excellent in terms of accuracy of visual inspection of the content and a prefilled syringe filled with a high viscosity drug that are suitable for injection of high viscosity drugs.

### BACKGROUND ART

In recent years, prefilled syringes prefilled with drugs have been used for reasons such as prevention of mistakes during medical treatment and prevention of bacterial contamination. A prefilled syringe has the tip opening of a barrel sealed with a cap member, is filled with a drug inside the barrel, has the rear end portion of the barrel sealed with a gasket, and is transported and stored in that state. When administering, an injection needle or an apparatus for administration is attached to the tip of the barrel, and by pushing a plunger attached to the gasket towards the tip and sliding the gasket inside the barrel, the drug flows out from the injection needle and is administered. As such, prefilled syringes have various advantages, such as allowing drugs to be administered in accurate doses without mistakes even during emergencies as there is no need to prepare the drugs at the point of treatment, being highly sanitary as there is no transferring of drugs, and being easy to operate.

Since prefilled syringes are stored and circulated in a state of being filled with a drug, it may be several years from the filling of the drug in production factories to administration. As such, while it goes without saying that long-term stability is needed, it is also necessary to be able to confirm the safety of the drug by visually inspecting for contamination by impurities. For that reason, the material constituting the barrel needs to be highly transparent, and barrels made of glass, which ensures transparency, have been frequently used in conventional prefilled syringes.

However, glass barrels crack relatively easily, need to be separated from the other parts and cannot be incinerated together therewith when discarded, and cost more, so there has been a demand for barrels made of resin. Resins with transparency comparable to that of glass barrels have appeared in recent years, and there has been a gradual transition towards resin barrels.

Regardless of the material of the barrel, to ensure sufficient slidability between the barrel and gasket, a lubricant layer composed of silicone or the like is generally provided on the inner peripheral surface of the barrel and/or the outer peripheral surface of the gasket.

In the case of conventionally used glass barrels, typically, silicone, in the form of an emulsion, is applied to the inner peripheral surface of the barrels and is fixed by baking at a high temperature (200 to 300°C). Silicone in itself is not harmful to the human body, but the silicone is fixed to the inner peripheral surface of the barrels to avoid the silicone contaminating the drugs.

In the case of resin barrels, since the glass transition point of resins is lower than the baking temperature of silicone, the same fixing treatment as for glass barrels cannot be used. In the case of resin barrels, methods in which a radiation or ultraviolet-curable organopolysiloxane is used and methods in which a photopolymerization catalyst such as benzophenone is added to silicone have been proposed as examples of methods for fixing silicone instead of baking at a high temperature (Patent Document 1).

On the other hand, as methods not involving such a fixing treatment, methods in which a silicone oil is simply applied to the inner peripheral surface of a barrel have also been widely used. In particular, in order to prevent the silicone oil from dripping from the inner peripheral surface of the barrel and contaminating the drug and to suppress increases in the sliding resistance of the gasket, the addition of a fine silica powder to a silicone oil has been proposed (Patent Document 2).

Additionally, in order to ensure sufficient slidability between the barrel and gasket, a prefilled syringe involving the use of a sealing stopper (gasket) for a syringe, which is a rubber stopper with its surface laminated with a tetrafluoroethylene resin film or an ultrahigh molecular weight polyethylene film, has also been proposed (Patent Document 3).

Patent Document 1: JP-A 2007-244606
Patent Document 2: JP-A 2006-94895
Patent Document 3: JP-A H10-314305

### SUMMARY OF THE INVENTION

However, since methods for the lubrication treatment of resin barrels comprising fixation require a step of curing by radiation etc. as described in the above Patent Document 1, production efficiency is inevitably poor. Additionally, some curing agents etc. may affect the human body when contaminating a drug.

On the other hand, when the fixing treatment is not performed, naturally, there is a risk of the applied silicone oil separating from the inner peripheral surface of the barrel during filling of a drug, storage or transport and contaminating the drug, causing turbidity. This is, as described in the above Patent Document 2, not a problem that can be completely overcome even when, for example, the silicone oil contains a fine silica powder. Rather, in that case, there is a risk of not only the silicone oil, but also the fine silica powder contaminating the drug.

Such contamination by the silicone oil from the inner peripheral surface of the barrel is particularly notable when the viscosity of the drug is high. While the exact mechanism is unclear, this is thought to be due to the high shear stress exerted on the silicone oil adhering to the inner peripheral surface of the barrel when filling the syringe with a drug of high viscosity. As mentioned above, silicone oil is not necessarily harmful to the human body, but it is not possible to clearly distinguish between turbidity caused by contamination due to silicone oil and turbidity caused by substantial contamination due to impurities by visual inspection alone, so such syringes may be determined to be defective products during inspection or medical practice and be forced to be discarded without ever being used.

Further, even when the silicone oil adheres normally to the inner peripheral surface of the barrel, the refractive index of the applied silicone oil differs from the refractive index of the drug and the refractive index of the synthetic resin constituting the syringe, resulting in glare on the inner peripheral surface of the barrel, which may interfere with visual inspection or make it seem as if there has been contamination by impurities or a defect such as a scratch on the barrel.

Moreover, in the case of the sealing stopper (gasket) for a syringe described in Patent Document 3, since the surface of the rubber stopper is laminated with a resin film, the error in the inner diameter of the sealing stopper (gasket) for a syringe or the barrel could be increased due to the disparity of the actual dimensions with respect to the dimensions of the original design, and there tended to be problems in the slidability or sealing properties of the sealing stopper (gasket) for a syringe with respect to the inner surface of the barrel.

As such, there has been a need for syringes capable of reducing the risk of separation and contamination by silicone oil while not requiring fixation of the silicone oil, in which glare rarely occurs on the inner peripheral surface of the barrel, and equipped with sufficient gasket slidability and sealing properties.

The present invention was achieved in view of the above circumstances, with an object of providing a syringe excellent in inspection accuracy while ensuring slidability and sealing properties between the barrel and gasket, and in particular, a syringe that is also suitable for filling with a high viscosity drug.

As a result of diligent studies, the present inventors found that by spraying a silicone oil of a predetermined kinematic viscosity onto the inner peripheral surface of a resin barrel at a predetermined application amount per unit area, it is possible to suppress separation and contamination by the silicone oil and glare on the inner peripheral surface of the barrel in addition to providing sufficient slidability.

That is, the syringe of the present invention is characterized by having a resin barrel, a gasket slidably inserted in the barrel, a plunger attached to the gasket, and a silicone film formed by applying a silicone oil having a kinematic viscosity of 500 to 100,000 cSt to the inner peripheral surface of the above-described barrel in an amount of 5 to 50 $\mu$g per 1 $cm^2$ of area.

Since a silicone oil having a kinematic viscosity of at least 500 cSt is used as the silicone constituting the silicone film in this syringe, when spraying the silicone oil, the silicone oil is appropriately maintained on the inner peripheral surface of the barrel without running. For that reason, even when a small amount of silicone oil is applied, it is possible to ensure sufficient slidability with the gasket. Additionally, since a silicone oil having a kinematic viscosity of at most 100,000 cSt is used, it can be applied to the inner peripheral surface of the barrel by spraying, and the silicone oil can be evenly applied in the above predetermined application amount per unit area.

Further, by using a silicone oil having a kinematic viscosity within that range, it is possible to ensure sufficient slidability between the barrel and gasket even when the amount of the silicone oil applied is at most 50 $\mu$g per 1 $cm^2$ of area on the inner peripheral surface of the barrel, and the amount of the silicone oil applied can be suppressed to a low amount. As a result thereof, when filling with a drug, even if the silicone oil becomes mixed into the drug, the amount of contamination can be kept extremely low. As such, the occurrence of turbidity due to contamination by the silicone oil can be suppressed, the causes of turbidity in a drug in a prefilled syringe can be limited to cases of contamination by impurities other than silicone oil, and accuracy in visual inspection to ensure safety can be substantially improved. This is particularly applicable to cases where a high viscosity drug which is susceptible to contamination by silicone oil is loaded. Further, when the application amount is within this range, as long as observation is performed by the naked eye, there is also a low likelihood of glare being detected on the inner peripheral surface of the barrel. Moreover, when the amount of the silicone oil applied to the inner peripheral surface of the barrel is at least 5 $\mu$g per 1 $cm^2$ area, sufficient slidability between the barrel and the gasket can be ensured.

Since the viscosity of a silicone oil having a kinematic viscosity within the above range is high, it is generally not easy to evenly spray the oil. However, even spraying is possible by appropriately adjusting the liquid temperature, air pressure, nozzle diameter and application time etc. In particular, a fine mist can be sprayed to achieve an extremely thin film such as one within the above range by heating the silicone oil within such a range as not to cause denaturation at the time of spraying.

Moreover, by designing the maximum outer diameter of the gasket to be greater than the inner diameter of the barrel such that the difference between the maximum outer diameter of the gasket and the inner diameter of the barrel is at least 0.02 mm and at most 0.50 mm, it is possible to suppress drug leakage from the gap between the gasket and barrel while maintaining the sealing properties of the gasket and ensure sufficient slidability between the barrel and gasket.

Further, as a result of diligent studies, it was found that when, upon shining incident light with an optical axis orthogonally intersecting the central axis of the barrel and measuring the angle of refraction from the optical axis of the transmitted light scattered along the same direction as the central axis, glare on the inner peripheral surface of the barrel can be remarkably suppressed if the angle of refraction is within a predetermined range.

That is, it was found that the glare could be remarkably suppressed when, upon shining an incident beam with a wavelength of 635 nm to 690 nm and a beam width of at most 3.0 mm on a barrel filled with a drug at an optical axis orthogonally intersecting the central axis of the barrel, the angle of refraction from the optical axis of the transmitted light scattered in the same direction as the above-described central axis was within a range of 0.1 to 0.5°.

The "angle of refraction" in the present invention refers to the aperture angle from the optical axis of transmitted light scattered along the same direction as the central axis of the barrel of a prefilled syringe filled with a drug when shining an incident beam with an optical axis orthogonally intersecting the central axis of the barrel.
The barrel of a prefilled syringe will cause a transmitted beam in a direction perpendicular to the central axis to be highly refracted with the center of curvature as the central axis. Accordingly, refraction occurring in the direction perpendicular to the central axis is affected by solely the shape of the barrel, and cannot indicate small variations in the application state of the silicone oil on the inner peripheral surface of the barrel. On the other hand, as the barrel is not substantially curved in the direction of the central axis, the divergence from the optical axis occurring in the same direction as the central axis, i.e. the "angle of refraction" in the present invention, is not significantly affected by the shape of the barrel, and can directly reflect the state of application of the silicone oil.

It was found that when the angle of refraction of a prefilled syringed filled with a drug is within the range of 0.1 to 0.5°, as long as the observation is performed by the naked eye, there is an extremely low likelihood of glare being detected on the inner peripheral surface of the barrel. As such, a prefilled syringe having such an angle of refraction can remarkably improve the visual inspection accuracy of the drug.

According to the present invention, a drug can be more stably stored in the barrel and the accuracy of inspection of the content can be substantially improved while ensuring the sealing properties and the slidability between the barrel and gasket. This makes safe and accurate operation possible. As such, the syringe according the present invention has great utility as a medical apparatus and as a cosmetic apparatus.

**BREIF DESCRIPTION OF THE DRAWINGS**

[Fig. 1] A schematic view of a prefilled syringe according to an embodiment of the present invention.
[Fig. 2] A schematic view showing an embodiment of a device for measuring an "angle of refraction" in the present invention.

**Description of Reference Numbers**

1    Prefilled syringe
10   Syringe
20   Barrel
21   Tip opening
22   Flange
23   Screw thread portion
24   Gasket
25   Plunger
26   Cap member
27   Drug
28   Silicone film
31   Laser oscillator
32   Projection plate
33   Incident beam
34   Transmitted beam

40    Central axis
41    Optical axis
42    Projection image

## MODES FOR CARRYING OUT THE INVENTION

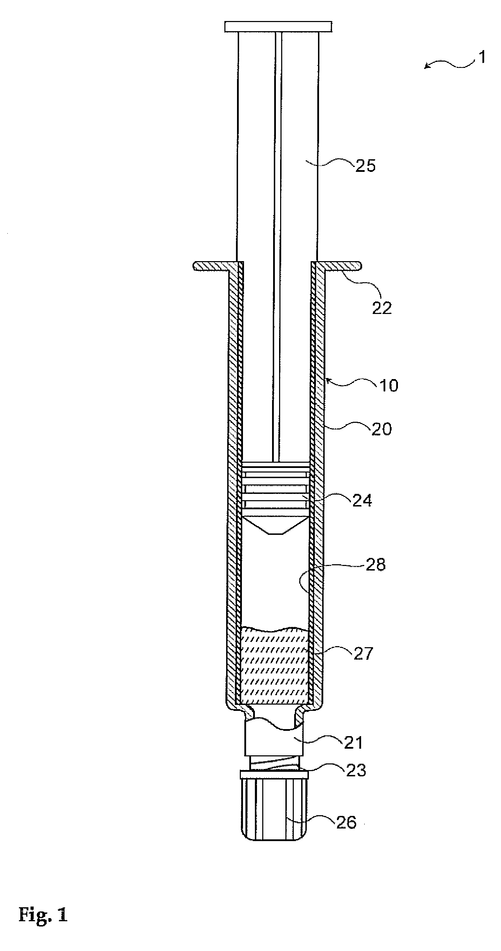

Herebelow, preferred embodiments of the present invention shall be explained in detail with reference to the attached drawings. Fig. 1 is a schematic view of a prefilled syringe which is a preferred embodiment of the present invention.

Prefilled syringe 1 according to the present embodiment can basically adopt the constitution of a conventional prefilled syringe as is, and as shown in Fig. 1, is constituted by a syringe 10 comprising a barrel 20 with a tip opening 21 at the tip, a liquid-tight, air-tight and slidable gasket 24 in barrel 20, and a plunger 25 attached to the rear end of gasket 24; a cap member 26 for sealing tip opening 21 of barrel 20; and a drug 27 stored inside syringe 10. Moreover, a silicone film 28 formed by spraying a silicone oil is provided on the inner peripheral surface of barrel 20. In Fig. 1, for the sake of illustration, silicone 28 is shown as a film seemingly applied at a fixed thickness, but as long as the amount of silicone oil applied to the inner peripheral surface of barrel 20 is within the range of 5 to 50 $\mu$g per 1 cm$^2$ area, the desired effects can be sufficiently achieved, so it does not necessarily need to be even.

<Barrel>

Barrel 20, as shown in Fig. 1, is a cylindrical body provided with tip opening 21 at the tip for the attachment of an injection needle, and a pair of opposing flanges 22 at the rear end for the placement of fingers during drug injection.
Additionally, the below-described sealing member, cap member 26, is attached to tip opening 21 of barrel 20. Moreover, an injection needle (not shown) instead of cap member 26 may be directly attached. In the present embodiment, a screw thread portion 23 is provided on the outer peripheral surface of tip opening 21 for attaching cap member 26 or an injection needle.
Barrel 20 is formed with a transparent resin material in order to enable visual inspection of the filled drug 27. While there is no particular limitation to the material forming barrel 20, when considering optical transparency, strength and dimensional accuracy, various resins, for example, polystyrenes, polyamides, polycarbonates, polyvinyl chloride, polyvinylidene chloride, poly-(4-methylpentene-1), polyvinyl alcohols, acrylic resins, acrylonitrile-butadiene-styrene copolymer, polyesters such as polyethylene terephthalate, cyclic polyolefins and cyclic olefin copolymers may be mentioned.
In the interest of visual inspection efficiency and accuracy of the content, cyclic olefin polymers (COP) and cyclic olefin copolymers (COC) which have excellent transparency are particularly preferred. As such resins, thermoplastic saturated norbornene resin compositions commercially available under Zeonex (trademark) from the (Japan) Zeon Corporation, particularly those dispersed with a compounding agent such as a gum polymer that is immiscible with the thermoplastic saturated norbornene resin, are preferred. In particular, those having the following properties are most preferred.
Optical transparency: 92%
Refractive index: 1.53

<Gasket>

While there is no particular limitation to the material of gasket 24, in order to maintain air-tightness, it is preferably formed by an elastic body such as rubber or a thermoplastic elastomer. Among them, butyl rubber, which changes little in dimensions upon autoclave sterilization, is particularly preferred as the main ingredient. As the butyl rubber, a halogenated butyl halide that has been chlorinated or brominated in order to improve crosslinkability and adhesiveness etc. may be used. As long as the material is permitted to be used as a medical apparatus or has been conventionally used as a material for forming the gasket of a syringe, there is no particular limitation. Additionally while there is no particular limitation on the surface material of the gasket, from the aspect of cost reduction, for example, materials not surface-treated with a tetrafluoroetilylene resin film or ultra high molecular weight polyethylene film are preferred. Moreover, in order to further reduce the possibility of the gasket being stuck, a silicone oil may be applied to the surface of the gasket.
Gasket 24 preferably has a plurality of ridge portions (ring-shaped convex portions) as shown in Fig. 1. By having such a plurality of ridge portions and valley portions (ring-shaped concave portions) provided in between, the sliding area between gasket 24 and barrel 20 can be reduced, and therefore the sliding resistance between gasket 24 and barrel 20 can be reduced. Additionally, by having such a plurality of ridge portions and valley potions provided in between, drug 27 can be blocked at multiple stages, suppressing leakage of drug 27 from the gap between gasket 24 and barrel 20.
Moreover, the maximum outer diameter of gasket 24 preferably corresponds to the outer diameter of the first ridge portion closest to the tip among the plurality of ridge portions. This is because the first ridge portion closest to the tip among the plurality of ridge portions of gasket 24 is in fact directly in contact with drug 27, so by maximizing the outer diameter of this ridge portion, leakage of drug 27 from the gap between gasket 24 and barrel 20 can be effectively suppressed.

<Dimensional difference between barrel and gasket>

In syringe 10 of the present embodiment, the maximum outer diameter of gasket 24 needs to be greater than the inner diameter of barrel 20. By making the maximum outer diameter of gasket 24 greater than the inner diameter of barrel 20, leakage of drug 27 from the gap between gasket 24 and barrel 20 can be suppressed, and the sealing properties of gasket 24 can be maintained.

Additionally, in syringe 10 of the present embodiment, the difference between the maximum outer diameter of gasket 24 and the inner diameter of barrel 20 needs to be at least 0.02 mm and at most 0.50 mm. This is because by making the difference between the maximum outer diameter of gasket 24 and the inner diameter of barrel 20 at least 0.02 mm and at most 0.50 mm, leakage of drug 27 from the gap between gasket 24 and barrel 20 can be suppressed while maintaining the sealing properties of gasket 24, and sufficient slidability between barrel 20 and gasket 24 can be ensured.

Moreover, the difference between the maximum outer diameter of gasket 24 and the inner diameter of barrel 20 is preferably at least 0.10 mm, and more preferably at least 0.15 mm. This is because the greater this difference is, the easier it is to suppress drug 27 from leaking from the gap between gasket 24 and barrel 20. On the other hand, the difference between the maximum outer diameter of gasket 24 and the inner diameter of barrel 20 is preferably at most 0.40 mm and more preferably at most 0.35 mm. This is because the smaller this difference is, the better is the slidabillty between the barrel and gasket.

The tolerance (variability in dimensional accuracy of the actual product with respect to the designed dimensions) of the maximum outer diameter of gasket 24 after autoclave sterilization is preferably controlled be at most $\pm$ 0.10 mm, and is more preferably controlled to be at most $\pm$ 0.05 mm. This is because when the variability in dimensional accuracy of gasket 24 is within this range, it is stabilized by the entire syringe 10, and sufficient slidability and sealing properties of the gasket can be ensured.

On the other hand, the tolerance (variability in dimensional accuracy of the actual product with respect to the designed dimensions) of the inner diameter of barrel 20 is preferably controlled to be at most $\pm$ 0.10 mm, and is more preferably controlled to be at most $\pm$ 0.05 mm. This is because when the variability in dimension accuracy of barrel 20 is within this range, it is stabilized by almost the entire syringe 10, and sufficient slidability and sealing properties of the gasket can be ensured.

If gasket 24 is a structure in which a tetrafluoroethylene resin film or ultra high molecular weight polyethylene film is laminated on the surface of a rubber stopper, keeping the difference between the maximum outer diameter of gasket 24 and the inner diameter of barrel 20 within these ranges might be difficult. This is because when making a gasket 24 with such a complex laminated structure, the production process becomes complicated, and as a consequence thereof, there is a tendency for the disparity in the actual dimensions of gasket 24 with respect to the dimensions of the original design to be greater. For that reason, even if the inspection process of the dimensional accuracy of gasket 24 were applied strictly, the proportion of gaskets 24 outside the predetermined dimensional accuracy would be too great, stalling and lowering the production of gasket 24, the production costs would soar significantly, and too much a burden would be placed on the inspection process, so the actual construction of a production line could be difficult.

As such, in order to control such a highly accurate maximum outer diameter of gasket 24, in addition to improving the dimensional accuracy in the production process for both gasket 24 and barrel 20 or strictly applying the inspection process for dimensional accuracy, gasket 24 is preferably one that is not surface-treated with a resin film. This is because the structure of the gasket itself can be designed into a simple shape, and the production process of the gasket itself can be simplified by doing so. That is, in syringe 10 of the present embodiment, the highly accurate maximum outer diameter of gasket 24 is preferably controlled by improving the dimensional accuracy in the production process for both gasket 24 and barrel 20 or strictly applying the inspection process for dimensional accuracy in addition to using gasket 24 that is not surface-treated with a resin film.

<Plunger>

Additionally plunger 25 only needs to be equipped with a strength that can withstand the bending and pressing force required to make gasket 24 slide inside barrel 20, and may be made of, for example, a hard plastic material such as polyethylene or polypropylene, but as long as the material is permitted to be used as a medical apparatus or has been conventionally used as a material for forming the gasket of a syringe, there is no particular limitation.

<Cap member>

Cap member 26 tightly adheres to tip opening 21 of barrel 20, air-tight seals tip opening 21, and may be made using an elastic body or hard resin such as butyl rubber, high-density polyethylene, polypropylene, polystyrene, or polystyrene terephthalate, but as long as the material is permitted to be used as a medical apparatus or has been conventionally used as a material for forming the gasket of a syringe, there is no particular limitation. In the present embodiment, a female thread portion for threading thread portion 23 formed on the outer peripheral surface of tip opening 21 of barrel 20 is formed on the inner peripheral surface of cap member 26.

<Silicone film>

Silicone film 28 formed by spraying a silicone oil having a predetermined kinematic viscosity as described below is provided on the inner peripheral surface of barrel 20. Since the silicone oil applied to barrel 20 only needs to satisfy the predetermined application amount per unit area, the thickness of silicone film 28 does not necessarily need to be even across the entirety of barrel 20.

(Silicone oil)

While the silicone oil forming silicone film 28 applied to the inner peripheral surface of the barrel is basically polydimethylsiloxane, a polydimethylsiloxane with a side chain or terminal substitution within a range not impairing lubricity may be used. Specifically for example, polymethylphenylsiloxane and polymethylhydrogen siloxane may be mentioned. Various additives may be added to the silicone oil as necessary.

The above-described silicone oil preferably has a kinematic viscosity of 500 to 100,000 cSt at 25 °C, and in particular, one having a kinematic viscosity of 1,000 to 30,000 cSt is more preferably used. When the kinematic viscosity is at least 500 cSt, the silicone oil is appropriately maintained at the spraying site on the inner peripheral surface of barrel 20 without running from the inner peripheral surface of barrel 20, so the slidability between barrel 20 and gasket 24 can be sufficiently ensured with a small amount of application. Moreover, when the kinematic viscosity is at most 100,000 cSt, application to the inner peripheral surface of barrel 20 by spraying is possible.

(Thickness of silicone film)

The application amount of the silicone oil constituting silicone film 28 is preferably 5 to 50 $\mu$g, and particularly preferably 10 to 30$\mu$g, per 1 cm$^2$ of the inner peripheral surface of barrel 20.
If the application amount of the silicone oil is at least 5 $\mu$g per 1 cm$^2$ of the inner peripheral surface of the barrel, a sufficient slidability between barrel 20 and gasket 24 can be ensured. Moreover, if the application amount is at most 50 $\mu$g per 1 cm$^2$ of the inner peripheral surface of the barrel, even if the silicone oil is mixed into the drug when loading drug 27, the amount of contamination can be kept extremely small. Further, as long as observation is performed by the naked eye, glare will not be detected on the inner peripheral surface of barrel 20.

(Method for forming silicone film)

Silicone film 28 is formed by evenly spraying a silicone oil having the above-described kinematic viscosity on the inner peripheral surface of barrel 20 using a spray system compatible with high viscosity solutions. Since the silicone oil applied in the present invention has a high kinematic viscosity, liquid temperature, air pressure, nozzle diameter and application etc. need to be appropriately adjusted in order to be able to evenly spray the silicone oil on the inner peripheral surface of barrel 20.
Particularly, in the case of the above silicone oil of a high kinematic viscosity heating the silicone oil when spraying in particular makes the silicone oil easier to spray.

(Silicone oil applied to surface of gasket)

When spraying a silicone oil on the gasket, similar to applying a silicone oil to the inner peripheral surface of the barrel, a silicone oil having a kinematic viscosity of 500 to 100,000 cSt at 25 °C is preferably used, and in particular, one with a kinematic viscosity of 1,000 to 50,000 cSt is more preferably used. When the kinematic viscosity is at least 500 cSt, the applied silicone oil does not run and the lubricating action is maintained for a long period of time. Moreover, when the kinematic viscosity is at most 100,000 cSt, even application over the entire surface of the gasket is possible. As the method for application, a conventionally used method can be used, for example, a method in which the silicone oil is directly added to a tank containing the gasket and mixed or a method in which the gasket is mixed in water suspended with the silicone oil may be used.

(Amount of silicone oil applied to surface of gasket per unit area)

Since the application amount should be kept at the required minimum so as to suppress intermixture of the silicone oil into the drug even when applying the silicone oil to the gasket, the application amount of the silicone oil is preferably at most 0.3 mg and is more preferably at most 0.15 mg per 1 $cm^2$ of the surface area of the gasket

<Drug>

While there is no particular limitation to drug 27 as long as it can be loaded into a prefilled type syringe, syringe 10 of the above constitution is particularly suitable for loading a high viscosity drug. Usually, when a high viscosity drug is loaded, a high shear force is exerted on the inner peripheral surface of the barrel, so the silicone oil applied to the inner peripheral surface of the barrel is easily mixed into the drug, and as a result thereof, turbidity occurs easily. However, if a silicone oil of the above predetermined viscosity is applied to the inner peripheral surface of the barrel at the above predetermined application amount per unit area, the amount of contamination by the silicone oil can be kept extremely small. For that reason, syringe 10 of the above constitution can be considered to be particularly suitable for high viscosity drugs in which turbidity occurs easily.

Additionally, since the maximum value of the extrusion pressure during sliding is higher when using a high viscosity drug 27 as compared to cases where a low viscosity drug 27 is used, the tolerances of barrel 20 and gasket 24 must be made higher in order to suppress the maximum value of the extrusion pressure during sliding and to improve the operability of syringe 10. For that reason, syringe 10 of the above constitution can be considered to be particularly suitable for high viscosity drugs that tend to lead to higher maximum values of the extrusion pressure during sliding. Consequently, for example, syringe 10 of the above constitution allows stable storage of a high viscosity drug, even with a viscosity of approximately 60,000 mPa·s, and as such a drug, an aqueous solution of 1% high molecular weight sodium hyaluronate with a weight average molecular weight of 600,000 to 3,700,000 may be mentioned in particular.

<Angle of refraction measuring device>

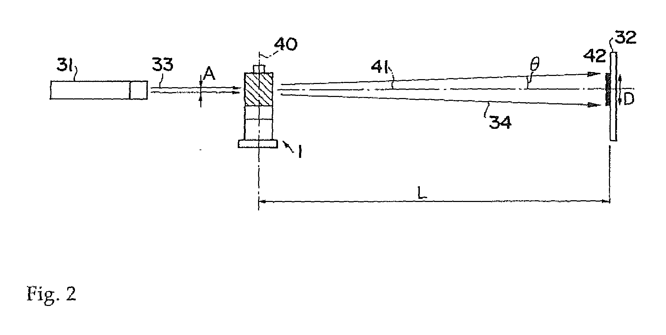

Fig. 2 is a schematic view showing an embodiment of a device for measuring an angle of refraction. This angle of refraction measuring device uses a laser oscillator 31 for shining a light beam (incident beam 33) on a prefilled syringe 1 and a projection plate 32 for projection of a light beam (transmitted beam 34) leaving prefilled syringe 1.

Laser oscillator 31 is a device for shining incident beam 33 of an optical axis 41 orthogonally intersecting the central axis 40 of barrel 20 onto prefilled syringe 1 filled with a drug. The wavelength of the oscillating laser is not particularly limited, and while a visible laser of any of red, green, blue and purple etc. may be used, the value of the angle of refraction changes with the wavelength, so the measurement needs to be carried out at a predetermined wavelength. As such, one within a wavelength range of 635 to 690 nm, which is that of common red lasers, is preferably used.

Projection plate 32 is not particularly limited as long as it is an opaque flat plate without any distortion on the surface. Projection plate 32 is arranged such that it is perpendicular to optical axis 41 of the light beam shone from laser oscillator 31.

<Method for measuring angle of refraction>

To measure the angle of refraction using the above device, the position of laser oscillator 31 is first fixed, then projection plate 32 is fixed such that it is perpendicular to the optical axis 41 of the light beam shone from laser oscillator 31. In this state, i.e. a state in which the object of measurement, prefilled syringe 1, is not positioned, the light beam shone from laser oscillator 31 is projected onto projection plate 32. When using a laser oscillator wherein the shape of a projection image 42 is more or less round, the diameter of the projection image 42 shall be considered to be the beam width "A" of incident beam 33. When using one that makes the shape of projection image 42 more or less oval, the direction of the laser oscillator is adjusted such that the direction of the short axis of the oval matches with the direction of the central axis of the barrel. In that case, the length of the short axis of the oval shall be considered to be the beam width "A" of incident beam 33. Additionally, laser oscillators making projection image 42 a shape other than round or oval are not suitable for measuring the angle of refraction in the present invention. Since it is more difficult to detect the difference in angle of refraction when the beam width "A" of incident beam 33 is large, it is preferably at most 3.0 mm and more preferably at most 2.0 mm.

Next, the object of measurement, prefilled syringe 1, is placed at a predetermined position on optical axis 41. At that time, the position of prefilled syringe 1 is adjusted such that the central axis 40 of prefilled syringe 1 orthogonally intersects optical axis 41.

In a state in which prefilled syringe 1 is arranged in the above manner, laser oscillator 31 shines a light beam (incident beam 33) on prefilled syringe 1, and transmitted beam 34 leaving prefilled syringe 1 is projected onto projection plate 32. The width "D" in the same direction as central axis 40 of projection image 42 projected on projection plate 32 and the distance "L" from central axis 40 of prefilled syringe 1 to projection plate 32 are measured.

The angle of refraction is an aperture angle "θ" from optical axis 41 of transmitted beam 34 scattered in the same direction as central axis 40 when shining incident beam 33 of optical axis 41 orthogonally intersecting central axis 40 of barrel 20 onto prefilled syringe 1 filled with a drug. Consequently, the angle of refraction can be obtained by the following formula using beam width "A" of incident beam 33 shone from laser oscillator 31, distance "L" from central axis 40 of prefilled syringe 1 to projection plate 32 and width "D" in the same direction as central axis 40 of projection image 42 of transmitted beam 34 projected on projection plate 32.

$$\text{Angle of Refraction } \theta = \tan^{-1}((D-A)/2L)$$

While embodiments of the present invention have been described with reference to the drawings above, they only serve to illustrate the present invention, and various constitutions other than the above may be adopted.
For example, in the above embodiments, the entire barrel 20 took the form of a single compartment filled with a drug, but the inside of barrel 20 may be separated into multiple compartments using at least one sealing stopper to achieve the form of a multi-compartment syringe. In that case, drug contamination and leakage can be more certainly prevented, and multiple drugs can be loaded into a single syringe.

**Examples**

Herebelow, the present invention shall be further explained using examples, but the present invention is not limited thereto.

<Example 1>

On the inner peripheral surface of a 5 ml volume barrel that was formed with a COP resin as the main ingredient, had a cylindrical outer diameter of 15.05 mm, a cylindrical inner diameter of 12.45 mm and a full length of 79.0 mm, a silicone oil of a kinematic viscosity of 5,000 cSt ("KF-96-5000cs" manufactured by Shin-Etsu Chemical Co., Ltd.) was sprayed under the following conditions such that the average application amount was 18 μg within a range of 12 to 25 μg per 1 cm$^2$. A thermoplastic saturated norbornene resin composition commercially available as Zeonex (trademark) from the (Japan) Zeon Corporation was used as the COP resin.
(Silicone oil spraying conditions)
Spraying time: 0.05 second
Air pressure: 0.5 MPa
Silicone oil heating temperature: 180 °C
Nozzle diameter: 1.0 mm

<Change in light transmittance due to formation of a silicone film>

Other than not spraying the silicone oil, a barrel similar to that of Example 1 (Comparative Example 1) was prepared, and compared with the barrel of the above Example 1 for light transmittance. The following device and method were used to measure light transmittance.

(Device)

- Spectrophotometer (manufactured by Hitachi High-Technologies Corporation; Model No.: U-3310)
- Wavelength: 660 nm

(Method)

9

- align the 0 point of the spectrophotometer in a state where nothing is in the sample chamber of the spectrophotometer.
- fix the barrel to the sample chamber of the spectrophotometer. At this time, keep the distance from the light source to the barrel constant, and adjust the light beam to shine on a position 20 mm from the tip of the barrel on the central axis of the barrel.
- read the absorptance value in a state where nothing is in the control cell holder.

The measurement results are shown in Table 1 below.

[Table 1]

| Sample | Measurement | Measured Value | Average |
|---|---|---|---|
| Example 1 (with silicone oil application) | 1st | 0.008 | 0.006 |
| | 2nd | 0.006 | |
| | 3rd | 0.005 | |
| Comparative Example 1 (without silicone oil application) | 1st | 0.007 | 0.007 |
| | 2nd | 0.009 | |
| | 3rd | 0.006 | |

As shown in the above Table 1, even when a silicone film was formed under the conditions described in Example 1, the absorptance did not appear to change substantially as compared to the case where silicone oil was not applied. Accordingly, the silicone film formed under the conditions of Example 1 was confirmed to not affect the efficiency of visual inspection.

<Example 2>

A prefilled syringe was assembled by preparing a barrel on which a silicone film was formed by the same method as Example 1, and attaching a cap member to this barrel, then filling 2.9 ml of an aqueous solution of 1% high molecular weight sodium hyaluronate with a weight average molecular weight of 3,000,000 (viscosity = 25,000 mPa·s), and capping it with a gasket.

<Example 3>

Other than using a silicone oil with a kinematic viscosity of 1,000 cSt ("KF-96-1000cs" manufactured by Shin-Etsu Chemical Co., Ltd.) as the silicone oil, a barrel on which a silicone film was formed was prepared in the same manner as Example 1, and a prefilled syringe was assembled using this barrel in the same manner as Example 2.

<Example 4>

Other than using a silicone oil with a kinematic viscosity of 30,000 cSt ("KF-96H-30000cs" manufactured by Shin-Etsu Chemical Co., Ltd.) as the silicone oil, a barrel on which a silicone film was formed was prepared in the same manner as Example 1, and a prefilled syringe was assembled using this barrel in the same manner as Example 2.

<Example 5>

A silicone oil was further applied to the surface of the gasket at 0.1 mg per 1 $cm^2$ of the surface. Specifically, a silicone oil with a kinematic viscosity of 5,000 cSt was added to a tank filled with water in an amount that would achieve 0.13 mg per 1 $cm^2$ with respect to the total surface area of the entire gasket, and mixed for 10 minutes to disperse it. A gasket was put into the tank, and after mixing for 10 minutes at 100°C while blowing a vapor from the bottom, the water was drained, rinsing was performed and autoclave sterilization was carried out. The application amount of the silicone oil was confirmed by gravimetry, and verified to be 0.10 mg per 1 $cm^2$ of the gasket surface. Other than using this gasket, a prefilled syringe was assembled in the same manner as Example 2.

<Example 6>

A silicone oil was further applied to the surface of the gasket at 0.2 mg per 1 cm$^2$ of the surface. While the application method was the same as Example 5, the silicone oil was added in an amount that would achieve 0.26 mg per 1 cm$^2$ with respect to the total surface area of the entire gasket. The application amount of the silicone oil was confirmed in the same manner as Example 5, and was 0.20 mg per 1 cm$^2$ of the gasket surface. Other than using this gasket, a prefilled syringe was assembled in the same manner as Example 2.

Comparative Example 2>

Other than using a silicone oil with a kinematic viscosity of 350 cSt ("KP-96-350cs" manufactured by Shin-Etsu Chemical Co., Ltd.) as the silicone oil, a barrel on which a silicone film was formed was prepared in the same manner as Example 1, and a prefilled syringe was assembled using this barrel in the same manner as Example 2.

<Comparative Example 3>

Other than using a mixed silicone oil with a kinematic viscosity of 150,000 cSt prepared by mixing 370 g of a silicone oil with a kinematic viscosity of 300,000 cSt ("KF-96-300000cs" manufactured by Shin-Etsu Chemical Co., Ltd.) and 630 g of a silicone oil with a kinematic viscosity of 100,000 cSt ("KF-96-100000cs" manufactured by Shin-Etsu Chemical Co., Ltd.), a barrel on which a silicone film was formed was prepared in the same manner as Example 1, and a prefilled syringe was assembled using this barrel in the same manner as Example 2.

<Comparative Example 4>

Other than spraying a silicone oil to form a silicone film with an average application amount of 100 μg per 1 cm$^2$, a barrel on which a silicone film was formed was prepared in the same manner as Example 1, and a prefilled syringe was assembled using this barrel in the same manner as Example 2.

<Comparative Example 5>

A silicone oil was further applied to the surface of the gasket at 0.4 mg per 1 cm$^2$ of the surface. While the application method is the same as Example 5, the silicone oil was added in an amount that would achieve 0.52 mg per 1 cm$^2$ with respect to the total surface area of the entire gasket. The application amount of the silicone oil was confirmed in the same manner as Example 5, and was 0.41 mg per 1 cm$^2$ of the gasket surface. Other than using this gasket, a prefilled syringe was assembled in the same manner as Example 2.
The prefilled syringes prepared in the above Examples 2 to 6 and Comparative Examples 2 to 5 were evaluated by the following methods for intermixture of silicone oil into the drug, glare on the inner peripheral surface of the barrel and sliding resistance.

<Visual evaluation>

The presence of turbidity in the drugs and the presence of glare on the inner peripheral surface of the barrels were visually evaluated by a group of five panelists consisting of skilled quality inspectors. The results are shown in Table 2. Turbidity evaluation criteria:

    A (good): no turbidity confirmed.
    B (poor): turbidity confirmed.

Glare evaluation criteria:

    A (good): no glare observed.
    B (poor): glare observed.

<Sliding resistance evaluation>

Injection needles (23G x 1 ¼; manufactured by Terumo Corporation) were affixed to the tip of the prefilled injection needles, and initial pressure and extrusion pressure when discharging the drugs at an extrusion speed of 100 mm/min. were measured using a testing machine ("EZ-TEST" manufactured by Shimadzu Corporation). Additionally, for the initial pressure measurement, samples stored for a month at 40 °C after production were used. The results are shown in Table 2.

Initial pressure evaluation criteria:

AA (best): local pressure maximum when gasket starts moving not confirmed in data less than 5 mm from the start of compression.

A (good): local pressure maximum when gasket starts moving 30 N or lower in data less than 5 mm from the start of compression.

B (poor): local pressure maximum when gasket starts moving over 30 N in data of less than 5 mm from the start of compression.

Extrusion pressure evaluation criteria:

A (good): dispersion in extrusion pressure within 5 N and extrusion pressure maximum 30 N or lower in data 5 mm or greater from the start of compression.

B (poor): dispersion in extrusion pressure over 5 N and extrusion pressure maximum over 30 N in data 5 mm or greater from the start of compression.

[Table 2]

| | Example 2 | Example 3 | Example 4 | Example 5 | Example 6 |
|---|---|---|---|---|---|
| Kinematic viscosity of silicone oil applied on barrel (cSt) | 5,000 | 1,000 | 30,000 | 5,000 | 5,000 |
| Average application amount per 1 $cm^2$ of silicone oil on barrel (μg) | 18 | 18 | 18 | 18 | 18 |
| Kinematic viscosity of silicone oil applied on gasket (cSt) | Not applied | Not applied | Not applied | 5,000 | 5,000 |
| Average application amount per 1 $cm^2$ of silicone oil on gasket (μg) | 0 | 0 | 0 | 0.10 | 0.20 |
| Turbidity | A | A | A | A | A |
| Glare | A | A | A | A | A |
| Initial pressure after one month storage | A | A | A | AA | AA |
| Extrusion pressure | A | A | A | A | A |

| | Comparative Example 2 | Comparative Example 3 | Comparative Example 4 | Comparative Example 5 |
|---|---|---|---|---|
| Kinematic viscosity of silicone oil applied on barrel (cSt) | 350 | 150,000 | 5,000 | 5,000 |
| Average application amount per 1 $cm^2$ of silicone oil on barrel (μg) | 18 | 0-3000 In the form of unsprayable droplets | 100 | 18 |
| Kinematic viscosity of silicone oil applied on gasket (cSt) | 0 | 0 | 0 | 5,000 |
| Average application amount per 1 $cm^2$ of silicone oil on gasket (μg) | 0 | 0 | 0 | 0.41 |
| Turbidity | B | A | B | B |
| Glare | A | B | B | A |
| Initial pressure after one month storage | A | B | A | A |
| Extrusion pressure | B | B | A | A |

As shown in the above Table 2, the prefilled syringes on which a silicone film was formed satisfying the predetermined conditions according to the invention (Examples 2 to 6) were observed to have no silicone oil contaminating the drugs and no glare on the inner peripheral surface of the barrels, and they exhibited excellent properties in terms of slidability. In particular, when the silicone oil was also applied at the predetermined amount to the surface of the gaskets, the initial pressure could be suppressed so much that a local maximum was not observed.

On the other hand, when the kinematic viscosity of the silicone oil was too low (Comparative Example 2), while it was fine in terms of glare, the sliding resistance was unstable and some turbidity in the drug, i.e. incorporation of silicone oil, was observed. Moreover, when the kinematic viscosity of the silicone oil was too high (Comparative Example 3), it could not be evenly sprayed in the form of fine particulates, and adhered unevenly as droplets with diameters ranging from several hundred $\mu$m to several mm, as a consequence of which there were portions where it was not applied and portions where it was excessively applied, and glare, and unstable sliding resistance were confirmed.

Further, even if the kinematic viscosity was within the optimal range, when the application amount of the silicone oil was too high (Comparative Example 4), glare was confirmed on the inner peripheral surface of the barrel, and intermixture of the silicone oil in the drug was observed. Additionally, when the amount of silicone oil applied to the surface of the gasket was too high (Comparative Example 5), intermixture of the silicone oil into the drug was observed.

<Example 7>

On the inner peripheral surface of a 5 ml volume barrel formed with a COP resin as the main ingredient, and produced and inspected by controlling the tolerance of the outer diameter to 15.05 $\pm$ 0.1 mm, the inner diameter to 12.45 $\pm$ 0.05 mm, the full length to 79.0 $\pm$ 0.2 mm, and the flange diameter 0 to 22.0 $\pm$ 0.2 mm, a silicone oil of a kinematic viscosity of 5,000 cSt ("KF-96" manufactured by Shin-Etsu Chemical Co., Ltd.) was sprayed under the following conditions to form a silicone film where the average application amount was 18 $\mu$g per 1 cm$^2$. A thermoplastic saturated norbornene resin composition commercially available as Zeonex (trademark) from the (Japan) Zeon Corporation was used as the COP resin. Additionally, during the production and inspection of the barrel, the tolerance control of the inner diameter ($\pm$ 0.05 mm) was particularly strictly observed.
(Silicone oil spraying conditions)
Spraying time: 0.05 second
Air pressure: 0.5 MPa
Silicone oil heating temperature: 180 °C
Nozzle diameter: 1.0 mm

On the other hand, a gasket made of butyl rubber, a kind of rubber material whose surface is not resin treated, was produced and inspected by controlling the tolerance of the outer diameter 0 to 12.70 $\pm$ 0.10 mm (first ridge portion), Ø to 12.0 $\pm$ 0.10 mm (valley portion) and full length to 10.0 $\pm$ 0.30 mm. Additionally, since a butyl rubber was used for this gasket, the dimensional changes due to autoclave sterilization were small as compared to general gaskets, and even when sterilized, would be kept within a tolerance range of $\pm$ 0.10 mm. Additionally, the gasket was applied with the silicone oil (KF-96-5000cs manufactured by Shin-Etsu Chemical Co., Ltd.) at 0.1 mg per 1 cm$^2$ area.

<Comparative Example 6>

A syringe was prepared in basically the same manner as Example 7 but differed in that a gasket of butyl rubber whose surface was laminated with a tetrafluoroethylene resin film, having a tolerance of an outer diameter Ø of 12.70 $\pm$ 0.10 mm (first ridge portion), Ø of 12.0 $\pm$ 0.10 mm (valley portion) and full length of 10.0 $\pm$ 0.30 mm was used and silicone oil was not applied.

Additionally, the gaskets of Example 7 and Comparative Example 6 were preliminarily checked for how the dimensions of the gaskets before autoclaving would change after autoclaving. The results revealed that there was almost no dimensional change in the gasket of Example 7 consisting of butyl rubber whose surface was not resin treated after autoclaving as compared to before autoclaving, and dimensional accuracy was kept within the tolerance range (data not shown). On the other hand, it was revealed that there was a large dimensional change in the gasket of Comparative Example 6 made of butyl rubber whose surface was laminated with a tetrafluoroethylene resin film after autoclaving as compared to before autoclaving, and the dimensional accuracy for the outer diameter Ø became 12.70 $\pm$ 0.20 mm (first ridge portion), Ø 12.0 $\pm$ 0.20 mm (valley portion) and full length 10.0 $\pm$0.40 mm after autoclaving.

<Comparative Example 7>

A syringe was prepared in basically the same manner as Example 7 but differed in that a 5 ml volume barrel formed with a COP resin as the main ingredient, and produced and inspected by controlling the tolerance of the outer diameter to 15.05 $\pm$ 0.1 mm, the inner diameter to 12.45 $\pm$ 0.20 mm, the full length to 79.0 $\pm$ 0.2 mm and the flange diameter Ø to 22.0 ± 0.2 mm was used.

<Slidability and air-tightness tests>

Since the most lenient combined tolerances in Example 7 for the barrel inner diameter Ø is 12.50 mm and for the first ridge portion of the gasket Ø is 12.60 mm, the difference between them is 0.10 mm. Even in that case, when real liquid leakage tests were performed as described below, the ability to secure air-tightness was confirmed. On the other hand, since the tightest combined tolerances in Example 7 for the barrel inner diameter Ø is 12.40 mm and for the first ridge portion of the gasket Ø is 12.80 mm, the difference between them is 0.40 mm. Even in that case, a good slidability was confirmed (data not shown).

<Real liquid leakage test>

In order to confirm that the drug solution would not leak from the gaps in the gasket even when a certain degree of pressure was applied, real liquid leakage tests were performed in accordance with the following steps.

1) affix an injection needle sealed at the tip onto the syringe.
2) push the plunger rod using an extrusion tester (EZ-TEST manufactured by Shimadzu Corporation), adjust the position of the pusher such that the extrusion pressure will be within a range of 19 to 24 N, and keep for 30 seconds.
3) remove the syringe, and visually confirm whether the drug solution has leaked from the gap of the gasket.

According to the results, in the case of Example 7, real liquid leakage tests were performed n = 50 times, but there was no sample where drug solution leakage occurred, so the ability to secure air-tightness was confirmed.

<Initial pressure and maximum pressure tests>

In the following sliding resistance test, injection needles (23 G x 1 ¼; manufactured by Terumo Corporation) were affixed to the tip of the syringes and plungers were affixed thereto, the extrusion pressures when compressing the plungers at a speed of 100 mm/min. were measured using an extrusion tester (EZ-TEST manufactured by Shimadzu Corporation). Table 3 shows the results of measurements of the initial pressure (extrusion pressure at a peak appearing within 5 mm from the start of compression) in ten syringes of each sample in a state not filled with drug solutions. Table 4 shows the results of measuring the maximum pressure (maximum value of the extrusion pressure) in 10 syringes of each sample filled with drug solutions.
[Table 3]

| Sample | Average value of initial pressure at each number of days of storage (N) | | |
|---|---|---|---|
| | 7 days | 30 days | 90 days |
| Example 7 | 4.0 | 4.4 | 5.3 |
| Comparative Example 6 | 11.5 | 12.1 | 13.7 |
| Comparative Example 7 | 13.7 | 16.1 | 16.2 |

[Table 4]

| Sample | Average value of maximum pressure at each number of days of storage (N) | | |
|---|---|---|---|
| | 7 days | 30 days | 90 days |
| Example 7 | 23.4 | 23.8 | 23.1 |
| Comparative Example 6 | 25.9 | 25.6 | 24.5 |
| Comparative Example 7 | 31.0 | 31.8 | 32.1 |

As shown above, when comparing Example 7 and Comparative Example 7 in Table 3, it is clear that the control of gasket tolerances greatly improved the initial pressure (locking). Additionally, from Table 4, it is clear that the control of gasket tolerances also improved the maximum pressure.

<Tests for comparing slidability in samples with different viscosities>

After filling the syringes of Example 7 and Comparative Example 7 with solutions of different viscosities and storing them at 40 °C for one month, slidability was measured and compared (same gaskets). The test results are shown in Table 5.

[Table 5]

| Syringe used | Loaded liquid | Viscosity of loaded liquid (mPa·s) | Maximum pressure (N) |
|---|---|---|---|
| Example 7 | 1% hyaluronic acid solution (weight average molecular weight 3,000,000) | 25000 | 23.8 |
| | 1% hyaluronic acid solution (weight average molecular weight 800,000) | 1800 | 21.4 |
| | Water | 1 | 5.1 |
| Comparative Example 7 | 1 % hyaluronic acid solution (weight average molecular weight 3,000,000) | 25000 | 31.8 |
| | 1% hyaluronic acid solution (weight average molecular weight 800,000) | 1800 | 27.8 |
| | Water | 1 | 11.2 |

As shown in Table 5, the maximum pressure changes greatly with the viscosity of the drug solution. As such, it is clear that the higher the viscosity of a drug solution, the greater the need to control the tolerances in order to suppress the maximum pressure.

<Example 8 to 13 and Comparative Examples 8 to 12>

he inner peripheral surface of a 5 ml volume barrel that was formed with a COP resin as the main ingredient, had a cylindrical outer diameter of 15.05 mm, a cylindrical inner diameter of 12.45 mm and a full length of 79.0 mm, was sprayed with a silicone oil of a kinematic viscosity of 5,000 cSt ("KF-96-5000cs" manufactured by Shin-Etsu Chemical Co., Ltd.) under the following conditions to be within a range of 0 to 150 $\mu$g per 1 $cm^2$. A thermoplastic saturated norbornene resin composition commercially available as Zeonex (trademark) from the (Japan) Zeon Corporation was used as the COP resin.

(Silicone oil spraying conditions)
Spraying time: 0.05 second
Air pressure: 0.5 MPa
Silicone oil heating temperature: 180 °C
Nozzle diameter: 1.0 mm

A prefilled syringe was assembled by attaching a cap member to this barrel, then loading 2.9 ml of an aqueous solution of 1% high molecular weight sodium hyaluronate with a weight average molecular weight of 3,000,000 (viscosity = 25,000 mPa·s), and capping it with a gasket coated with the same silicone oil as above at 0.10 mg per 1 $cm^2$.

<Angle of refraction measurement>

To measure the angle of refraction of the above prefilled syringe, as shown in Fig.2, the following device, conditions and method were used.

(Device)

- Laser oscillator: RX-4N (manufacture by Sakura Color Products Corp. Japan)
- Beam width ("A") of light beam shone from laser oscillator: 2 mm
- Wavelength: 650 nm
- Output: less than 1 mW

(Conditions)

- Distance from laser oscillator to central axis of prefilled syringe: 50 mm
- Distance ("L") from central axis of prefilled syringe to projection plate: 200 mm
- Site of incidence: center of the region in the barrel filled with the drug on the central axis of the barrel

(Method)

- for the portion filled with the drug in each prefilled syringe, measure the width ("D") in the direction of the central axis of the image projected on the projection plate in three installments by rotating 120° each time with the central axis as the rotation axis, and calculate the average.
- based on the obtained width of projection ("D"), the optical width of the light beam shone ("A"), and the distance from the central axis of the prefilled syringe to the projection plate ("L"), calculate angle of refraction θ.

<Glare evaluation>

The presence of glare on the inner peripheral surface of the barrel of each prefilled syringe was visually evaluated by a group of five panelists consisting of skilled quality inspectors.
Glare evaluation criteria:

A (good): no glare observed.
B (poor): glare observed.

<Sliding resistance evaluation>

The sliding resistance between the barrel and gasket of each prefilled syringe was evaluated using the following criteria.

AA (best): local pressure maximum when gasket starts moving not confirmed, and no variation in extrusion pressure after gasket starts moving.
A (good): pressure when gasket starts moving being within the permitted range, and no variation in extrusion pressure after gasket starts moving.
B (poor): pressure when gasket starts moving being within the permitted range, but variations present in extrusion pressure after gasket starts moving.

The measurement and evaluation results are shown in Table 6 below.

[Table 6]

|  | Application amount of silicone oil ($\mu$g/cm$^2$) | Projection image width (D) (mm) | Angle of Refraction θ (°) | Glare | Sliding resistance |
|---|---|---|---|---|---|
| Example 8 | 5 | 3.0 | 0.14 | A | A |

(continued)

|  | Application amount of silicone oil ($\mu$g/cm$^2$) | Projection image width (D) (mm) | Angle of Refraction $\theta$ (°) | Glare | Sliding resistance |
|---|---|---|---|---|---|
| Example 9 | 10 | 3.0 | 0.14 | A | A |
| Example 10 | 20 | 3.0 | 0.14 | A | AA |
| Example 11 | 30 | 3.7 | 0.24 | A | AA |
| Example 12 | 40 | 4.3 | 0.33 | A | AA |
| Example 13 | 50 | 5.0 | 0.43 | A | AA |
| Comparative Example 8 | 0 | 3.0 | 0.14 | A | B |
| Comparative Example 9 | 60 | 6.7 | 0.67 | B | AA |
| Comparative Example 10 | 80 | 7.3 | 0.76 | B | AA |
| Comparative Example 11 | 100 | 9.0 | 1.00 | B | AA |
| Comparative Example 12 | 150 | 10.3 | 1.19 | B | AA |

As shown in the above Table 6, no glare was observed on the inner peripheral surfaces of barrels of the prefilled syringes with angles of refraction within a range of 0.1 to 0.5° (Examples 8 to 13), and they exhibited excellent slidability. On the other hand, glare was confirmed on the inner peripheral surface of the barrel when the angle of refraction exceeded the range of 0.1 to 0.5° (Comparative Examples 9 to 12). Additionally, when a silicone oil was not applied to the inner peripheral surface of the barrel (Comparative Example 8), no glare was confirmed, but sliding resistance was confirmed to be unstable.

The present invention has been explained with reference to examples above. These examples are only exemplifications, and those skilled in the art will recognize that various modifications are possible, and that such modifications are also within the scope of the present invention.

## Claims

1. A syringe having a resin barrel, a gasket slidably inserted in the barrel, a plunger attached to the gasket, and a silicone film formed by applying a silicone oil having a kinematic viscosity of 500 to 10,000 cSt to the inner peripheral surface of said barrel in an amount of 5 to 50 $\mu$g per 1 cm$^2$ of area.

2. The syringe according to claim 1, **characterized in that** a silicone oil having a kinematic viscosity of 500 to 10,000 cSt is applied to the surface of said gasket at 0 to 0.3 mg per 1 cm$^2$ of area.

3. The syringe according to claim 1 or 2, wherein the tolerance of the inner diameter of said barrel is controlled to be at most $\pm$ 0.10 mm.

4. The syringe according to any one of claims 1 to 3, **characterized in that** said barrel consists of a thermoplastic saturated norbornene resin composition.

5. The syringe according to any one of claims 1 to 4, wherein said gasket has a maximum outer diameter greater than the inner diameter of said barrel, and the difference between the maximum outer diameter of said gasket and the inner diameter of said barrel is at least 0.02 mm and at most 0.50 mm.

6. The syringe according to claim 5, wherein said gasket has a plurality of ridge portions, the outer diameter of a first ridge portion closest to the tip among said plurality of ridge portions corresponding to said maximum outer diameter.

7. The syringe according to any one of claims 1 to 6, wherein the tolerance of the maximum outer diameter of said gasket after autoclave sterilization is controlled to be $\pm$ 0.10 mm

8. The syringe according to any one of claims 1 to 7, wherein said gasket consists of a rubber or thermoplastic elastomer.

9. The syringe according to claim 8, wherein said rubber is butyl rubber.

10. The syringe according to any one of claims 1 to 9, wherein when shining an incident beam with a wavelength of 635 nm to 690 nm and a beam width of at most 3.0 mm on the barrel filled with a drug with an optical axis orthogonally intersecting the central axis of the barrel, the angle of refraction from the optical axis of a transmitted beam scattered in the same direction as said central axis is within a range of 0.1 to 0.5°.

11. The syringe according to any one of claims 1 to 10, which is a prefilled syringe having a cap member sealing the tip opening of said barrel and a drug loaded inside said barrel.

12. The syringe according to claim 11, **characterized in that** said drug has a viscosity of 1,000 to 60,000 mPa·s.

13. The syringe according to claim 11 or 12, wherein said drug is an aqueous sodium hyaluronate solution.

**Fig. 1**

Fig. 2

**INTERNATIONAL SEARCH REPORT**

| | International application No. |
|---|---|
| | PCT/JP2009/070285 |

A.  CLASSIFICATION OF SUBJECT MATTER
*A61M5/28*(2006.01)i

According to International Patent Classification (IPC) or to both national classification and IPC

B.  FIELDS SEARCHED

Minimum documentation searched (classification system followed by classification symbols)
A61M5/28

Documentation searched other than minimum documentation to the extent that such documents are included in the fields searched
Jitsuyo Shinan Koho        1922–1996   Jitsuyo Shinan Toroku Koho   1996–2010
Kokai Jitsuyo Shinan Koho   1971–2010   Toroku Jitsuyo Shinan Koho   1994–2010

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| X<br>Y | JP 10-201844 A  (Yugen Kaisha Koki Enjiniaringu),<br>04 August 1998 (04.08.1998),<br>claim 3; paragraph [0035]<br>(Family: none) | 1,3,7,8,10<br>2,4-6,9,<br>11-13 |
| Y | JP 2004-321614 A  (Terumo Corp.),<br>18 November 2004 (18.11.2004),<br>all drawings<br>(Family: none) | 2,11-13 |

[X]  Further documents are listed in the continuation of Box C.          [ ]  See patent family annex.

| * Special categories of cited documents: | |
|---|---|
| "A"  document defining the general state of the art which is not considered to be of particular relevance | "T"  later document published after the international filing date or priority date and not in conflict with the application but cited to understand the principle or theory underlying the invention |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
|---|---|
| 04 March, 2010 (04.03.10) | 16 March, 2010 (16.03.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

21

## INTERNATIONAL SEARCH REPORT

| | International application No. |
|---|---|
| | PCT/JP2009/070285 |

C (Continuation).　DOCUMENTS CONSIDERED TO BE RELEVANT

| Category* | Citation of document, with indication, where appropriate, of the relevant passages | Relevant to claim No. |
|---|---|---|
| Y | JP 6-197965 A  (Nippon Zeon Co., Ltd.), 19 July 1994 (19.07.1994), paragraphs [0039], [0047] to [0049] & US 5561208 A (specification, column 10, lines 10 to 16; column 11, lines 35 to 62) & EP 0559146 A1 (specification, page 8, lines 11 to 14; column 9, lines 10 to 26) | 4 |
| Y | JP 2003-180832 A  (Hori Garasu Kabushiki Kaisha), 02 July 2003 (02.07.2003), claim 1; paragraph [0030] (Family: none) | 5,6 |
| Y | JP 2007-244606 A  (Hisamitsu Pharmaceutical Co., Inc.), 27 September 2007 (27.09.2007), paragraphs [0019], [0035] (Family: none) | 9,12,13 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007244606 A
- JP 2006094895 A
- JP H10314305 A